# EUROPEAN PATENT SPECIFICATION

(11) **EP 1 870 426 B1**
(45) Date of publication and mention of the grant of the patent: **27.10.2010**
(21) Application number: 05818716.2
(22) Date of filing: 09.12.2005
(51) Int. Cl.: C08J 5/02, C08L 7/02, C08L 9/10, A41D 19/00, A61F 6/04

(54) **ANTIALLERGIC PRODUCTION OF LATEX OR PVC AND METHOD FOR FORMING THE SAME**
ANTIALLERGISCHE HERSTELLUNG VON LATEX ODER PVC UND FORMGEBUNGSVERFAHREN DAFÜR
PRODUIT EN LATEX OU PVC ANTIALLERGIQUE ET MÉTHODE POUR LE FORMER

(30) Priority: 15.04.2005 CN 05138895; 16.05.2005 CN 05140025
(43) Date of publication of application: 26.12.2007
(73) Proprietor: Zhou, Zhengming, Zhangjiagang Jiangsu 215600 (CN)
(72) Inventor: ZHOU, Zhengming 1406, Guotai Building, Jiangsu 215600 (CN); JIANG, Yan, Zhangjiagang City Jiangsu Province 215600 (CN)
(74) Representative: Howard, Paul Nicholas
(86) International application number: PCT/CN2005/002137
(87) International publication number: WO 2006/108333

(56) References cited:
- WO-A1-00/50507
- CN-A- 1 352 593
- CN-A- 1 557 351
- JP-A- 11 106 569
- US-A- 4 879 118
- US-A1- 2002 061 929
- US-A1- 2003 212 167
- DATABASE WPI Week 200336 Thomson Scientific, London, GB; AN 2003-376302 XP002508809 -& JP 2003 027083 A (OKAMOTO CO LTD) 29 January 2003 (2003-01-29)
- DATABASE WPI Week 200107 Thomson Scientific, London, GB; AN 2001-054067 XP002508815 -& JP 2000 308519 A (ERUBU KK) 7 November 2000 (2000-11-07)
- DATABASE WPI Week 199114 Thomson Scientific, London, GB; AN 1991-099140 XP002508810 -& JP 03 044323 A (KAO CORP) 26 February 1991 (1991-02-26)

## Description

### Technical Field

The present invention relates to an antiallergic latex or PVC product, condom or glove.

### Background Art

Natural latex is widely used in modem industries. Latex glove and condom, for instance, are made essentially of latex. Nevertheless, natural latex products can cause hypersensitivity to users due to the existence of residues such as soluble proteins in the latex raw materials; chemicals added during manufacture such as stabilizer acetaldehyde, accelerant tetramethylthiuram, and carbamate; as well as some natural pollutants produced during the process of latex acquirement.

Since natural latex glove is superior to gloves made of other materials in terms of strength, elasticity, and comfortability, it has been widely applied in different areas for the past few years. However, gloves made from natural latex can also cause allergy, which is a serious problem for glove-users, with the symptoms including the appearance of red spot on skin, itchy, inflammation, etc. Currently in China, US, and European countries, methods used to reduce the occurrence of skin allergy all involve cutting down protein content, among which enzymatic breakdown is the one used most often to lower the quantity of soluble proteins in latex. Nevertheless, in natural latex, proteins function as stabilizers. If protein content goes down, the quality of the glove will also go down. Furthermore, the process of protein removal is sophisticated and not cost-effective (Zhao ke, Rubber industry, pages 679-81, No. 11, volume 51, 2004).

Presently, alternatives are also adopted to solve the problem. For instance, artificial latex or PVC (polyvinyl chloride) was used to replace natural latex in glove manufacture. Although allergy problem (caused by impurities such as protein) can be solved through this way, gloves made from artificial latex require complex techniques and the cost is very high. In addition, these gloves are airtight, longtime wearing will also cause allergy-like symptoms such as itchy among users. For allergy prone population, it is especially easy to develop symptoms such as perspiration, itchy, and inflammation.

Using condom is one of the most popular and simplest ways for male contraceptive purposes. It has been well-accepted by countries all over the world for its convenience, high efficiency and comfortability. With the rapid spread of AIDS, condom becomes one of the most effective tools for preventing sexually transmitted diseases. Presently, most condoms are made of latex. The existence of residues such as soluble proteins in the latex raw materials, chemicals added during manufacture, and some natural pollutants produced during the process of latex acquirement may cause allergic symptoms to some condom users. For male user, the typical allergic symptoms include glans rubiosis, the appearance of papules, itchy, sting pain, etc. In serious situations, the symptoms also include ulceration and erosion, which may further cause infection; For female user, the typical allergic symptoms include itchy or burning feelings around external genital or in vagina, congestion of vagina mucous membrane, edema, and leucorrhea increase (TianYin, Population and Birth Control, 2000, No.4:52). One major solution to relieve the symptoms is to orally take or locally administrate antiallegic medicines, but this has caused much inconvenience to condom users, which severely limits its application.

### Description of the Invention

Therefore, it is an object of the present invention to provide an antiallergic latex or PVC glove or condom. By incorporating natural antiallergic medicines into its matrix, the latex or PVC glove or condom possesses superior antiallergic function.

By incorporating natural antiallergic medicines into its matrix, the glove both possesses superior antiallergic function and is comfortable to wear, especially suitable for an allergy prone person or a frequent glove-wearer.

By incorporating natural antiallergic medicines into its matrix, the condom overcomes the side-effects caused by allergy, and the condom user will no longer have allergic symptoms. As a result, the condom of the present invention can be applied to a broader range of population.

Since natural medicines function mildly and have low side effects, they are generally used as additives for food and health products. By incorporating natural antiallergic medicines into its matrix, the latex or PVC product of the present invention solves the problem of easy to cause allergy. In addition, the manufacture process of the antiallergic latex or PVC product is simple and cost-effective, suitable for large-scale production.

Specifically, the natural antiallergic medicines added in the condom of the present invention have antibacterium and antivirus effect, which can prevent the potential bacterium or virus contamination caused by long-term storage, making it safer to wear.

By incorporating natural antiallergic medicines into its matrix, the glove of the present invention overcomes the discomfort and allergic symptoms. In addition, the natural antiallergic medicines have antibacterium and antivirus effect, which can prevent the potential bacterium or virus contamination caused by long-term storage, making it safer to wear.

The present invention provides an antiallergic latex or PVC glove or condom, which comprises in its matrix at least one natural antiallergic medicines selected from baicalin, matrine, arctigenin, oleanolic acid, tetrandrine, sodium ferulate, total alkaloid of asiatic moonseed rhizome, ginkgo bilobal A, total glycoside of Rhizoma Anemones flaccidae, magnolin, and osthole.

The natural antiallergic medicines added in the latex or PVC condom or glove of the present invention is preferably matrine and/or sodium ferulate.

In the latex or PVC product of the present invention, the content of the natural antiallergic medicines is generally 0.001%∼1% by weight, and preferably, 0.005%∼0.5% by weight.

The present invention further provides a method for producing a latex or PVC product, which comprises the step of adding the natural antiallergic medicines into the matrix of the latex of PVC raw materials.

In one preferred embodiment of the present invention, a condom is provided, which contains in its matrix the natural antiallergic medicines.

The natural antiallergic medicine is at least one selected from baicalin, matrine, arctigenin, oleanolic acid, tetrandrine, sodium ferulate, total alkaloid of asiatic moonseed rhizome, ginkgo bilobal A, total glycoside of Rhizoma Anemones flaccidae, magnolin and, osthole, wherein matrine and/or sodium ferulate are preferred.

The matrix of the condom is preferably latex, i.e., the condom is a latex condom.

The content of the natural antiallergic medicines in the condom is preferably 0.001%∼1% by weight, and more preferably, 0.005%∼0.5% by weight.

Generally, the optimum weight ratio of the medicine is in the range of 0.011%∼0.016%, with the content of the medicines being 0.25mg∼0.39mg per condom.

The manufacture process of the condom of the present invention is simple and suitable for large-scale production. Compared to conventional condom-making process, what is needed is just to mix the medicines with other regular ingredients.

The process is described in details as follows: centrifuging the gel materials, formulating, adding medicines, maturing in storage, importing into an elevated tank, mold cleaning, drying, dipping in gel, gumming, drying at 100-110°C for about 50 minutes, gumming again, drying at 60-100°C for about 50 minutes, curling, drying at 100-130°C for about 50 minutes, demoulding, bath-washing at 50-60°C for about 30 minutes, drying at 120°C for about 1 hour, oiling and then packaging.

Since the medicines will suffer partial loses in gumming and drying steps, the quantity of the medicines added during preparation should be slightly higher than the content of the medicines contained in the final product. Generally, the content of the medicines in the condom of the present invention is about 50% to 80% of the amount added during preparation.

By incorporating in its matrix the natural antiallergic medicines, the condom of the present invention is both safe and effective. Several preferred natural antiallergic medicines have been widely used in clinics, and the effectiveness of these medicines has also been widely approved. Among these medicines, extraction of cortex dictamni radics and extraction of engelhardtia chryolepis can be aqueous extract or alcohol extract.

The latex or PVC glove of the present invention contains in its matrix the natural antiallergic medicines, wherein the latex includes both natural latex and artificial latex.

Through tremendous experiments, the inventor of the present invention found that the natural antiallergic medicine is preferably at least one selected from baicalin, matrine, arctigenin, oleanolic acid, tetrandrine, sodium ferulate, total alkaloid of asiatic moonseed rhizome, ginkgo bilobal A, total glycoside of Rhizoma Anemones flaccidae, magnolin, and osthole. When these medicines are used, the glove made by the present invention not only achieved a better antiallergic effect, but also obtained a better quality, while the color of the glove remained unchanged. Among these preferred medicines, matrine and/or sodium ferulate are even more preferred.

Based on the total weight of the glove, the content of the above-mentioned natural antiallergic medicines is in the range of 0.001%∼1% by weight, and more preferably, 0.005%∼0.5% by weight.

Specifically, the glove of the present invention can be produced by the following method: mixing the natural antiallergic medicines with latex or PVC materials, followed by routine latex or PVC glove-making procedures.

Since the medicines will suffer partial loses under high temperature treatment, the quantity of the medicines added during preparation is not equal to the content of the medicines contained in the final product. Generally, the content of the medicines in the glove of the present invention is about 10% to 20% of the amount added during preparation.

The gloves of the present invention were randomly chosen and cut into filaments with a length of 0.5∼1.0cm. The filaments were heated at 50°C in a 1000ml water solution for 30 minutes. The solution was then concentrated to a volume of 50ml. The content of the medicines in the glove was analyzed by HPLC, the result of which indicated that the average content of the medicines falls into the scope of the above-mentioned preferred ranges.

Over 100 subjects have tried on the gloves containing different medicines or different contents of the medicines for more than 5 hours, but no symptoms such as itchy or inflammation were reported, which indicates the glove of the present invention has antiallergic function.

### Mode for Carrying out the Invention

### Example 1

The mold was cleaned, soaked with demoulding agent at 35°C, and dried at 80°C. 20g matrine, 30g sodium ferulate, and 50g latex were mixed and the mold was immersed in it, followed by drying, chlorinating, washing with clean water, demoulding, heating at 90°C for 30 minutes, washing, chlorinating, cleaning, and drying at 100°C for 1 hour to obtain 3,500 gloves. The result of content assay indicated that the content of matrine is 0.021% by weight, and the content of sodium ferulate is 0.025% by weight.

The gloves produced by this example were worn by 50 subjects for 5 hours, and no symptoms such as itchy were reported, which indicates the glove of the present invention has superior antiallergic function. In the control group, common latex gloves were worn by another 50 subjects for 5 hours, and two itchy cases were reported.

### Example 2

The mold was cleaned, soaked with demoulding agent at 35°C, and dried at 80°C. 20g baicalin, 20g matrine, 30g tetrandrine, and 50g latex were mixed and the mold was immersed in it, followed by drying, chlorinating, washing with clean water, demoulding, heating at 90°C for 30 minutes, washing, chlorinating, cleaning, and drying at 100°C for 1 hour to obtain 3,500 gloves. The result of content assay indicated that the content of matrine is 0.021% by weight, the content of baicalin is 0.019% by weight, and the content of tetrandrine is 0.026% by weight.

The gloves produced by this example were worn by 50 subjects for 5 hours, and no symptoms such as itchy were reported, which indicates the glove of the present invention has superior antiallergic function. In the control group, common latex gloves were worn by another 50 subjects for 5 hours, and one itchy case was reported.

### Example 3

The mold was cleaned, soaked with demoulding agent at 35°C, and dried at 80°C. 30g ginkgo bilobal A, 30g oleanolic acid, 30g sodium ferulate, and 50g latex were mixed and the mold was immersed in it, followed by drying, chlorinating, washing with clean water, demoulding, heating at 90°C for 30 minutes, washing, chlorinating, cleaning, and drying at 100°C for 1 hour to obtain 3,500 gloves. The result of content assay indicated that the content of ginkgo bilobal A is 0.022% by weight, the content of oleanolic acid is 0.024% by weight, and the content of sodium ferulate is 0.025% by weight.

The gloves produced by this example were worn by 100 subjects for 5 hours, and no symptoms such as itchy were reported, which indicates the glove of the present invention has superior antiallergic function. In the control group, common latex gloves were worn by another 100 subjects for 5 hours, and two itchy cases were reported.

### Example 4. Preparation of PVC gloves (Reference Example)

The mold was cleaned, soaked with demoulding agent at 35°C, and dried at 80°C. The mold was immersed in 50kg PVC solution (PVC powders were mixed with POD and soybean oil while stirring to form gel). The gel was dried, chlorinated, washed by clean water, and demoulded. The resultants were soaked in a 50L solution containing 30g liquorice saponin at 50°C for 20 minutes, and then washed. After further subjected to the treatment of heating at 90°C for 30 minutes, washing, chlorinating, cleaning, and drying at 100°C for 1 hour, 3,000 gloves were obtained. The result of content assay indicated that the content of liquorice saponin in the glove is 0.013% by weight.

The gloves produced by this example were worn by 100 subjects for 5 hours, and no symptoms such as itchy were reported, which indicates the glove of the present invention has superior antiallergic function. In the control group, common latex gloves were worn by another 100 subjects for 5 hours, and one itchy case was reported.

### Example 5. Preparation of PVC gloves (Reference Example)

The mold was cleaned, soaked with demoulding agent at 35°C, and dried at 80°C. The mold was immersed in 50kg PVC solution (PVC powders were mixed with POD and soybean oil while stirring to form gel). The gel was dried, chlorinated, washed by clean water, and demoulded. The resultants were soaked in a 50L solution containing 30g matrine at 50°C for 30 minutes, and then washed. After further subjected to the treatments of heating at 90°C for 30 minutes, washing, chlorinating, cleaning, and drying at 100°C for 1 hour, 3,000 gloves were obtained. The result of content assay indicated that the content of matrine in the glove is 0.016% by weight.

The gloves produced by this example were worn by 200 subjects for 5 hours, and no symptoms such as itchy were reported, which indicates the glove of the present invention has superior antiallergic function. In the control group, common latex gloves were worn by another 200 subjects for 5 hours, and one itchy case was reported.

### Example 6

50kg gel materials were centrifuged and formulated. 10g matrine and 10g sodium ferulate were then added, followed by maturing in storage, importing into an elevated tank, mold cleaning, drying, immersing in latex at room temperature, drying at 105°C for 50 minutes, immersing in latex again, drying at 80°C for 50 minutes, curling, drying at 120°C for 50 minutes, demoulding, bath-washing at 50°C for 30 minutes, drying at 120°C for 1 hour, oiling and then packaging.

The condom of the present invention was cut into filaments with a length of O.S-I.Ocm. The filaments were heated at 50°C in a 1000ml water solution for 30 minutes. The solution was then concentrated to a volume of 50ml. The content of the medicines in the glove was analyzed by HPLC, the result of which indicated that the average content of the medicines is 0.31mg per condom, equivalent to 0.014% by weight of the condom.

### Example 7

50 kg gel materials were centrifuged and formulated. 10g oleanolic acid and 10g baicalin were then added, followed by maturing in storage, importing into an elevated tank, mold cleaning, drying, immersing in latex at room temperature, drying at 105°C for 50 minutes, immersing in latex again, drying at 80°C for 50 minutes, curling, drying at 120°C for 50 minutes, demoulding, bath-washing at 50°C for 30 minutes, drying at 120°C for 1 hour, oiling and then packaging.

The condom of the present invention was cut into filaments with a length of 0.5∼1.0cm. The filaments were heated at 50°C in a 1000ml water solution for 30 minutes. The solution was then concentrated to a volume of 50ml. The content of the medicines in the glove was analyzed by HPLC, the result of which indicated that the average content of the medicines is 0.31mg per condom, equivalent to 0.014% by weight of the condom.

### Experimental Example 1. Antiallergic study on local administration to rabbit vagina mucous

Materials: Japan flap-eared healthy rabbit, female, weight >2.5kg

Method: The rabbits were randomly divided into 2 groups, 20 rabbits for each. An antiallergic condom produced in Example 6 of the present invention was placed and maintained in the vagina of each rabbit in Group 1 for an hour. For Group 2, a regular condom was employed to replace the antiallergic condom. The experiments were carried out for 10 consecutive days. On day 10, the rabbits were sacrificed. The vaginas of the rabbits were firstly cut open for visual inspection, and then removed and fixed in 10% formaldehyde solution, followed by embeding, sectioning, staining, and histological examining. For rabbits tested with the antiallergic condom of the present invention (Group 1), the results of the histology observation showed no significant difference, and the results of the pathological section observation indicated no obvious changes. In contrast, one case with mucosal edema and one case with slightly epithelium ulcer were reported among rabbits tested with regular condoms (Group 2). In addition, minor allergy was observed in some Group 2 rabbits during the pathological section observation.

### Experimental Example 2. Antiallergic study on local administration to rabbit vagina mucous

Materials: Japan flap-eared healthy rabbit, female, weight >2.5kg

Method: The rabbits were randomly divided into 2 groups, 20 rabbits for each. An antiallergic condom produced in Example 7 of the present invention was placed and maintained in the vagina of each rabbit in Group 1 for an hour. For Group 2, a regular condom was employed to replace the antiallergic condom. The experiments were carried out for 10 consecutive days. On day 10, the rabbits were sacrificed. The vaginas of the rabbits were firstly cut open for visual inspection, and then removed and fixed in 10% formaldehyde solution, followed by embeding, sectioning, staining, and histological examining. For rabbits tested with the antiallergic condom of the present invention (Group 1), the results of the histology observation showed no significant difference, and the results of the pathological section observation indicated no obvious changes. In contrast, one case with slightly epithelium ulcer was reported among rabbits tested with regular condoms (Group 2). In addition, minor allergy was observed in some Group 2 rabbits during the pathological section observation.

The results from the above experimental examples indicate the condom of the present invention has antiallergic function.

## Claims

1. An antiallergic latex or PVC condom or glove, comprising in its matrix at least one natural antiallergic medicines selected from baicalin, matrine, arctigenin, oleanolic acid, tetrandrine, sodium ferulate, total alkaloid of asiatic moonseed rhizome, ginkgo bilobal A, total glycoside of Rhizoma Anemones flaccidae, magnolin, and osthole, wherein the content of the natural antiallergic medicines in the condom or glove is 0.005%-0.5% by weight, the condom or glove is obtained by adding the natural antiallergic medicines into latex or PVC materials, followed by routine latex or PVC product-making procedures.

2. The latex or PVC condom or glove of claim 1, wherein the natural antiallergic medicines is matrine and/or sodium ferulate.

3. A method for producing the latex or PVC condom or glove of claim 1, comprising the step of adding the natural antiallergic medicines into the matrix of the latex or PVC raw materials.

## Patentansprüche

1. Antiallergisches Latex- oder PVC-Kondom oder antiallergischer Latex- oder PVC-Handschuh, das bzw. der in seiner Matrix zumindest ein natürliches antiallergisches Arzneimittel aufweist, das ausgewählt ist aus Baicalin, Matrin, Arctigenin, Oleanolsäure, Tetrandrin, Natriumferulat, Gesamtalkaloid des asiatischen Mondsamenwurzelstocks, Ginkgo bilobal A, Gesamtglycosid des Wurzelstocks von Anemones flaccidae, Magnolin und Osthol, wobei der Gehalt des natürlichen antiallergenen Arzneimittels in dem Kondom oder Handschuh 0,005 bis 0,05 Gew.-% beträgt, und wobei das Kondom oder der Handschuh durch Zugabe der natürlichen antiallergenen Arzneimittel in Latex- oder PVC-Materialien, gefolgt von Verfahren zur Herstellung von Latex- oder PVC-Produkten, erhalten werden.

2. Latex- oder PVC-Kondom oder -Handschuh nach Anspruch 1, wobei das natürliche antiallergene Arzneimittel Matrin und/oder Natriumferulat ist.

3. Verfahren zur Herstellung des Latex- oder PVC-Kondoms oder Handschuhs nach Anspruch 1, das den Schritt des Zugebens des natürlichen antiallergenen Arzneimittels in die Matrix des Latex- oder PVC-Rohmaterials aufweist.

## Revendications

1. Préservatif ou gant en PVC ou latex antiallergique comprenant dans sa matrice au moins un médicament antiallergique naturel choisi parmi la baicaline, la matrine, l'arctigénine, l'acide oléanolique, la tétrandrine, le férulate de sodium, un alcaloïde total de rhizome de ménisperme asiatique, le ginkgo biloba A, un glycoside total de Rhizoma Anemones flaccide, la magnoline et l'osthole, dans lequel la teneur en médicament antiallergique naturel dans le préservatif ou le gant est de 0,005 % à 0, 5 % en poids, le préservatif ou le gant étant obtenu en ajoutant le médicament antiallergique naturel dans les matériaux en latex ou PVC, suivi par des procédures de routine de préparation de produit en latex ou PVC.

2. Préservatif ou gant en PVC ou latex selon la revendication 1, dans lequel le médicament antiallergique naturel est la matrine et/ou le férulate de sodium.

3. Procédé de préparation du préservatif ou gant en PVC ou latex selon la revendication 1, comprenant l'étape consistant à ajouter le médicament antiallergique naturel dans la matrice des matériaux bruts en latex ou PVC.
